(19) Europäisches Patentamt
European Patent Office
Office européen des brevets

(11) Veröffentlichungsnummer: **0 101 597**
**B1**

(12) **EUROPÄISCHE PATENTSCHRIFT**

(45) Veröffentlichungstag der Patentschrift: **22.06.88**

(51) Int. Cl.⁴: **C 07 C 175/00**, C 07 F 7/18

(21) Anmeldenummer: **83107909.0**

(22) Anmeldetag: **10.08.83**

(54) **Verfahren zur Herstellung von Astaxanthin und Zwischenprodukten in der Astaxanthin-Synthese.**

(30) Priorität: **20.08.82 CH 4983/82**
**21.06.83 CH 3392/83**

(43) Veröffentlichungstag der Anmeldung:
**29.02.84 Patentblatt 84/9**

(45) Bekanntmachung des Hinweises auf die Patenterteilung:
**22.06.88 Patentblatt 88/25**

(84) Benannte Vertragsstaaten:
**AT BE CH DE FR GB IT LI NL**

(56) Entgegenhaltungen:
**DE - A - 2 653 838**

**TETRAHEDRON LETTERS, Nr. 49-50, 1974, Seiten 4319-4322, Pergamon Press, GB; G.M. RUBOTTOM et al.: "Peracid oxidation of trimethylsilyl enol ethers: A facile alpha-hydroxylation procedure"**
**JOURNAL OF ORGANIC CHEMISTRY, Band 43, Nr. 8, 1978, Seiten 1599-1602; G.M. RUBOTTOM et al.: "m-Chloroperbenzoic acid oxidation of 2-trimethylsilyloxy-1,3-dienes. Synthesis of alpha-hydroxy and alpha-acetoxy enones"**
**Revue Romaine de Chimie 22 (7) 1085-1088 (1977)**
**Helv. Chim. Acta 28, 427-436 (1945)**

(73) Patentinhaber: **F. HOFFMANN-LA ROCHE & CO. Aktiengesellschaft, CH-4002 Basel (CH)**

(72) Erfinder: **Bernhard, Kurt, Dr., Bündtenstrasse 11, CH-4411 Lupsingen (CH)**
Erfinder: **Müller, Robert Karl, Dr., Wettsteinallee 145, CH-4058 Basel (CH)**
Erfinder: **Spruijtenburg, Robert, Dr., Krummenrainweg 9, CH-4153 Reinach (CH)**

(74) Vertreter: **Lederer, Franz, Dr. et al, Van der Werth, Lederer & Riederer Patentanwälte Lucile-Grahn-Strasse 22, D-8000 München 80 (DE)**
Vertreter: **Cottong, Norbert A. et al, Grenzacherstrasse 124 Postfach 3255, CH-4002 Basel (CH)**

**EP 0 101 597 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein neues Verfahren zur Herstellung von Cyclohexenonderivaten, nämlich von Astaxanthin und Zwischenprodukten in der Astaxanthinsynthese. Die Erfindung betrifft ferner neue Zwischenprodukte in diesem Verfahren.

Tetrahedron Letters, 1974, Nr. 49–50, 4319–4322 und Journal of Organic Chemistry, Bd. 43, Nr. 8, 1978, 1599–1602 beschreiben die Überführung von Silylenoläthern von gesättigten bzw. α,β-ungesättigten Ketonen in Acyloine durch Persäureoxidation und anschliessende Hydrolyse. DE-A-2 653 838 offenbart die Herstellung von Astaxanthin aus gegebenenfalls verestertem 15,15'-Didehydro-astaxanthin. Die Dehydroverbindung wurde durch Wittig-Reaktion erhalten.

Das erfindungsgemässe Verfahren ist dadurch gekennzeichnet, dass man einen Silyl-enoläther der allgemeinen Formel

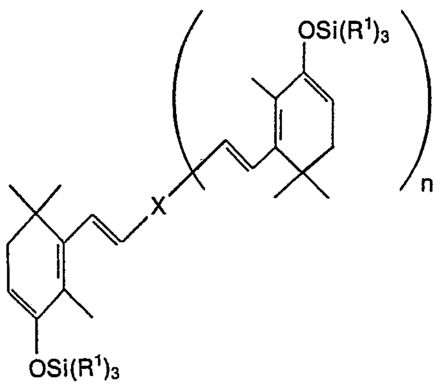

I

worin n für die Zahl 0 und X für eine Gruppe

$-C(CH_3)-CH=CH_2$
$R^2$

oder n für die Zahl 1 und X für die Gruppe 6,11-Dimethylhexadeca-2,4,6,8,10,12,14-heptaen-2,15-diyl

stehen, die Reste $R^1$ gleiche oder verschiedene Bedeutung haben und Alkylgruppen bezeichnen, und der Rest $R^2$ eine Trialkylsiloxygruppe $-OSi(R^1)_3$ oder eine Äthergruppe darstellt, mit Percarbonsäure oxidiert und, gewünschtenfalls, das erhaltene α-Siloxyketon der allgemeinen Formel

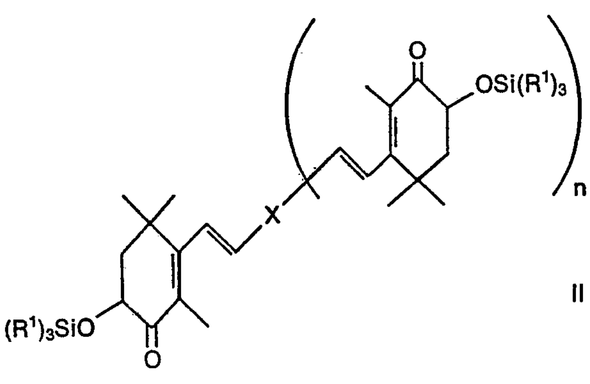

II

worin n, X und $R^1$ die obigen Bedeutungen haben, zu einem α-Hydroxyketon der allgemeinen Formel

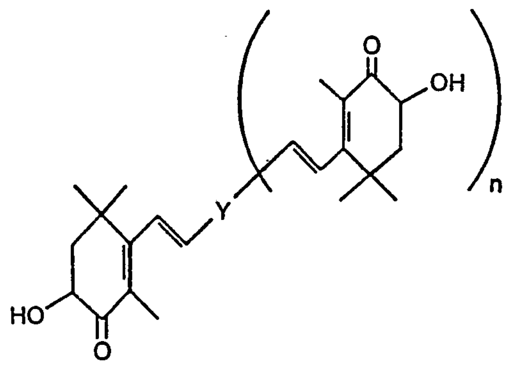

III

worin n für die Zahl 0 und Y für eine Gruppe

$-C(CH_3)-CH=CH_2$
$R^3$

oder n für die Zahl 1 und Y für die Gruppe 6,11-Dimethylhexadeca-2,4,6,8,10,12,14-heptaen-2,15-diyl

stehen, und $R^3$ die Hydroxygruppe oder eine Äthergruppe darstellt, hydrolysiert.

Der obige Ausdruck «Alkylgruppe» umfasst insbesondere Alkylgruppen mit 1 bis 20 Kohlenstoffatomen, wie Methyl, Äthyl, Propyl, Isopropyl, Butyl, Isobutyl, t-Butyl, Pentyl, Decyl, Octadecyl und dergleichen. Bevorzugte Alkylgruppen sind diejenigen, welche 1 bis 5 Kohlenstoffatome enthalten. Beispiele für Trialkylsiloxygruppen $-OSi(R^1)_3$ sind Triisopropylsiloxy, Octadecyl-dimethylsiloxy und insbesondere Trimethylsiloxy und t-Butyl-dimethylsiloxy.

Der Ausdruck «Äthergruppe» umfasst diejenigen Äthergruppen, welche üblicherweise zum Schutz einer Hydroxygruppe eingeführt werden. Bevorzugte Äthergruppen sind die Alkoxygruppen mit 1 bis 5 Kohlenstoffatomen und insbesondere die Methoxygruppe.

In den obigen Formeln I–III bezeichnet vorzugsweise n die Zahl 1 und X bzw. Y die Gruppe 6,11-Dimethylhexadeca-2,4,6,8,10,12,14-heptaen-2,15 diyl der Formel

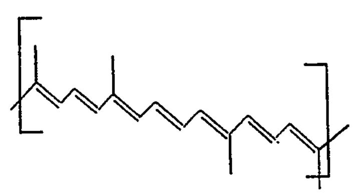

IV

Ferner bezeichnet in den obigen Formeln I–III vorzugsweise n die Zahl 0 und X bzw. Y die Gruppe

$-C(CH_3)-CH=CH_2$ .
$OCH_3$

Astaxanthin konnte bisher aus Canthaxanthin nur in kleiner Ausbeute via Astacin und Crustaxanthin [J. Chem. Soc. Chem. Commun. 49 (1967)] hergestellt werden. Das erfindungsgemässe Verfahren ermöglicht nun einen einfachen Zugang zu Astaxanthin aus Canthaxanthin oder aus Zwischenprodukten der Canthaxanthinsynthese.

Die Oxidation einer Verbindung der Formel I kann unter den Bedingungen erfolgen, welche üblicherweise bei einer Epoxidierung mittels Percarbonsäure angewendet werden. Hierbei findet vermutlich eine Epoxidierung der silylierten Enolgruppe und Öffnung des Epoxidringes unter 1,4-Wanderung der Silylgruppe statt. Beispiele geeigneter Percarbonsäuren sind Monoperphthalsäure, Peressigsäure, Perbenzoesäure, m-Chlorperbenzoesäure, p-Nitroperbenzoesäure, Permaleinsäure und dergleichen. Bevorzugte Percarbonsäuren sind Peressigsäure, Perbenzoesäure und insbesondere Monoperphthalsäure.

Durch die Anwesenheit grösserer Mengen Wasser oder Mineralsäure kann bereits während der Oxidation eine teilweise Hydrolyse des Reaktionsgemisches auftreten. Vorzugsweise wird daher eine Percarbonsäure verwendet, welche im wesentlichen frei von Wasser und Mineralsäure ist. Es kann jedoch auch mit «technischen» Percarbonsäuren gearbeitet werden, z.B. mit 40%-iger Peressigsäure. Zur Verringerung der Hydrolyseanfälligkeit kann in diesen Fällen zweckmässig ein Puffersalz zugesetzt werden, vorzugsweise ein Puffer mit einem pH-Wert von etwa 5–9, z.B. ein Acetatpuffer, ein Carbonatpuffer, ein Phosphatpuffer (pH 6) und dergleichen.

Um eine möglichst vollständige Umsetzung zu erreichen, wird zweckmässig mit einem Überschuss an Percarbonsäure gearbeitet. Bevorzugt werden etwa 1,2–5 Äquivalente und besonders bevorzugt etwa 1,6–3,0 Äquivalente Percarbonsäure pro silylierte Enolgruppe eingesetzt. Die erfindungsgemässe Oxidation wird zweckmässig in einem inerten organischen Lösungsmittel, beispielsweise einem Äther oder einem chlorierten oder aromatischen Kohlenwasserstoff, wie Diäthyläther, Tetrahydrofuran, Methylenchlorid, Toluol und dergleichen, durchgeführt. Bevorzugte Lösungsmittel sind die Äther, insbesondere Diäthyläther. Zur Erzielung einer hohen Selektivität wird vorzugsweise in verdünnter Lösung (z.B. 0,02–0,1 M Lösung der Verbindung der Formel I) gearbeitet und die Kontaktzeit mit der Percarbonsäure kurz gehalten (z.B. etwa 10–60 Minuten). Druck und Temperatur sind keine kritischen Aspekte in dieser Reaktion; vorzugsweise wird jedoch bei Atmosphärendruck und einer Temperatur von etwa −20 °C bis etwa +30 °C gearbeitet.

Die Hydrolyse der Verbindungen der Formel II kann nach den an sich bekannten Methoden der Hydrolyse von Silyläthern durchgeführt werden, beispielsweise durch Umsetzung mit verdünnten Mineralsäuren, wässrigen organischen Säuren, Trialkylammoniumfluorid und dergleichen oder durch Kochen in Methanol, gewünschtenfalls unter Zusatz einer Säure, z.B. p-Toluolsulfonsäure.

Die Verbindungen der obigen Formel I und II sind neu und bilden ebenfalls Gegenstand der vorliegenden Erfindung.

Die Verbindungen der Formel I können dadurch hergestellt werden, dass man eine Verbindung der allgemeinen Formel

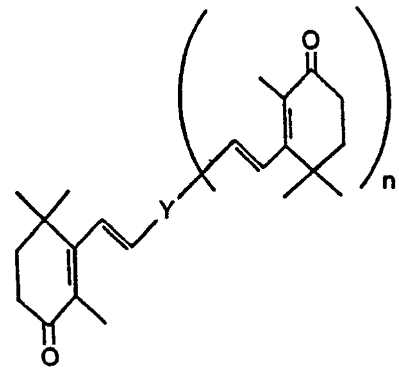

V

worin n und Y die obigen Bedeutungen haben, in einen Silyl-enoläther der Formel I überführt.

Dieses Verfahren ist neu. Es kann nach an sich bekannten Methoden [z.B. Liebigs Ann. Chem. 1981, 1643, Tetrahedron Letters 22, 3455 (1981), Synthesis 1979, 35] durchgeführt werden.

Eine bevorzugte Methode der Silylierung einer Verbindung der Formel V ist die Umsetzung einer Verbindung der Formel V mit einem Trialkylhalogensilan, z.B. Trimethylchlorsilan oder Trimethyljodsilan, in Gegenwart eines Alkalimetalldialkylamids, z.B. Lithium-diisopropylamid. Die Umsetzung wird vorzugsweise in einem Äther, z.B. Tetrahydrofuran, durchgeführt. Pro Carbonyl- und Hydroxygruppe in Formel V werden zweckmässig etwa 1,2–3,0 Äquivalente Silan und etwa 1,05–1,3 Äquivalente Dialkylamid, vorzugsweise etwa 2,0–2,4 Äquivalente Silan und etwa 1,2 Äquivalente Dialkylamid eingesetzt. Es hat sich ferner in vielen Fällen als vorteilhaft erwiesen, die Verbindung der Formel V, das Silan und das Lösungsmittel vorzulegen und dann das Dialkylamid zuzugeben. Temperatur und Druck dieser Reaktion sind nicht kritisch. Im allgemeinen wird jedoch bei Atmosphärendruck und einer Temperatur von etwa −40 °C bis etwa +30 °C, vorzugsweise bei etwa −15 °C gearbeitet.

Eine weitere Methode, welche insbesondere zur Umsetzung der Verbindungen der Formel V geeignet ist, worin n für die Zahl 0 steht, ist die Umsetzung mit einem Trialkylhalogensilan, z.B. Trimethylchlorsilan, in Gegenwart eines Trialkylamins, z.B. Triäthylamin. Diese Reaktion wird vorzugsweise in Dimethylformamid unter Rückfluss durchgeführt.

Eine weitere bevorzugte Methode ist ferner die Umsetzung einer Verbindung der Formel V mit einem Trifluormethansulfonsäuretrialkylsilylester, z.B. dem t-Butyl-dimethylsilylester, in Gegenwart eines Trialkylamins, z.B. Triäthylamin. Bevorzugtes Lösungsmittel ist Methylenchlorid. Druck und Temperatur sind nicht kritisch und im allgemeinen werden Atmosphärendruck und eine

Temperatur zwischen etwa −40 °C und Raumtemperatur, vorzugsweise etwa 0–10 °C angewendet.

Eine in Formel V gegebenenfalls vorhandene freie Hydroxygruppe wird im Gegensatz zu einer geschützten Hydroxygruppe bei diesen Umsetzungen ebenfalls silyliert. Durch Verwendung einer Äthergruppe kann daher der Verbrauch an Silylierungsmittel gesenkt werden. Die Einführung der Schutzgruppe kann nach den bekannten Methoden der Verätherung einer Hydroxygruppe erfolgen. Eine nachträgliche Abspaltung der Schutzgruppe erübrigt sich im allgemeinen, da diese bei der weiteren Umsetzung der Verbindung der Formel III B gemäss Schema 2 eliminiert wird.

Gemäss dem erfindungsgemässen Verfahren wird Astaxanthin in wenigen Stufen aus Canthaxanthin bzw. einem bekannten Zwischenprodukt der Canthaxanthinsynthese zugänglich. Schemata 1 und 2, worin $R^1$, $R^2$ und $R^3$ die obigen Bedeutungen haben, und X Chlor oder Brom bezeichnet, illustrieren einen einfachen Syntheseweg und verdeutlichen den Zusammenhang des Syntheseaufbaus für n=0 oder 1.

Schema 1

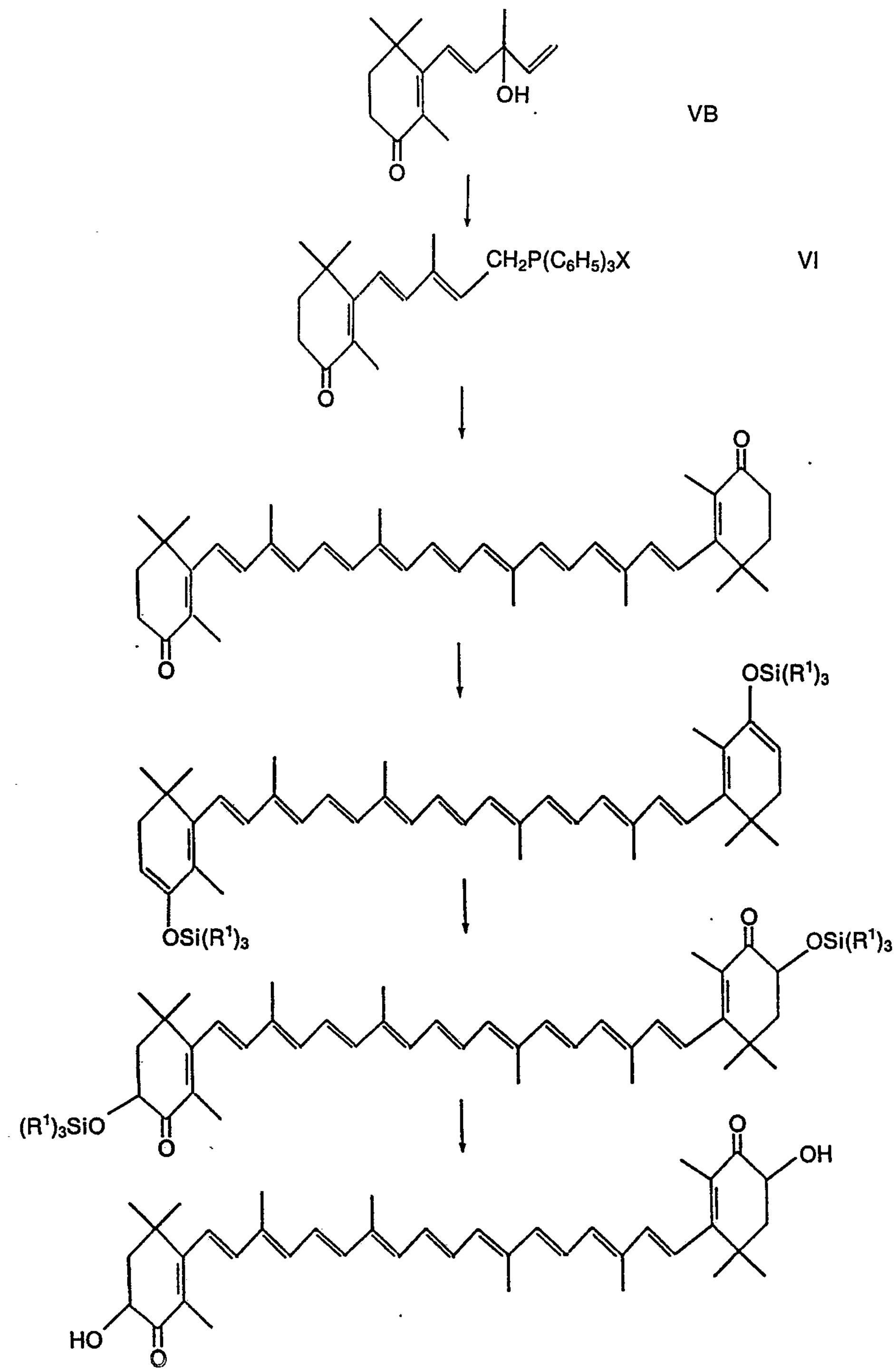

VB

VI

VA

IA

IIA

IIIA

Schema 2

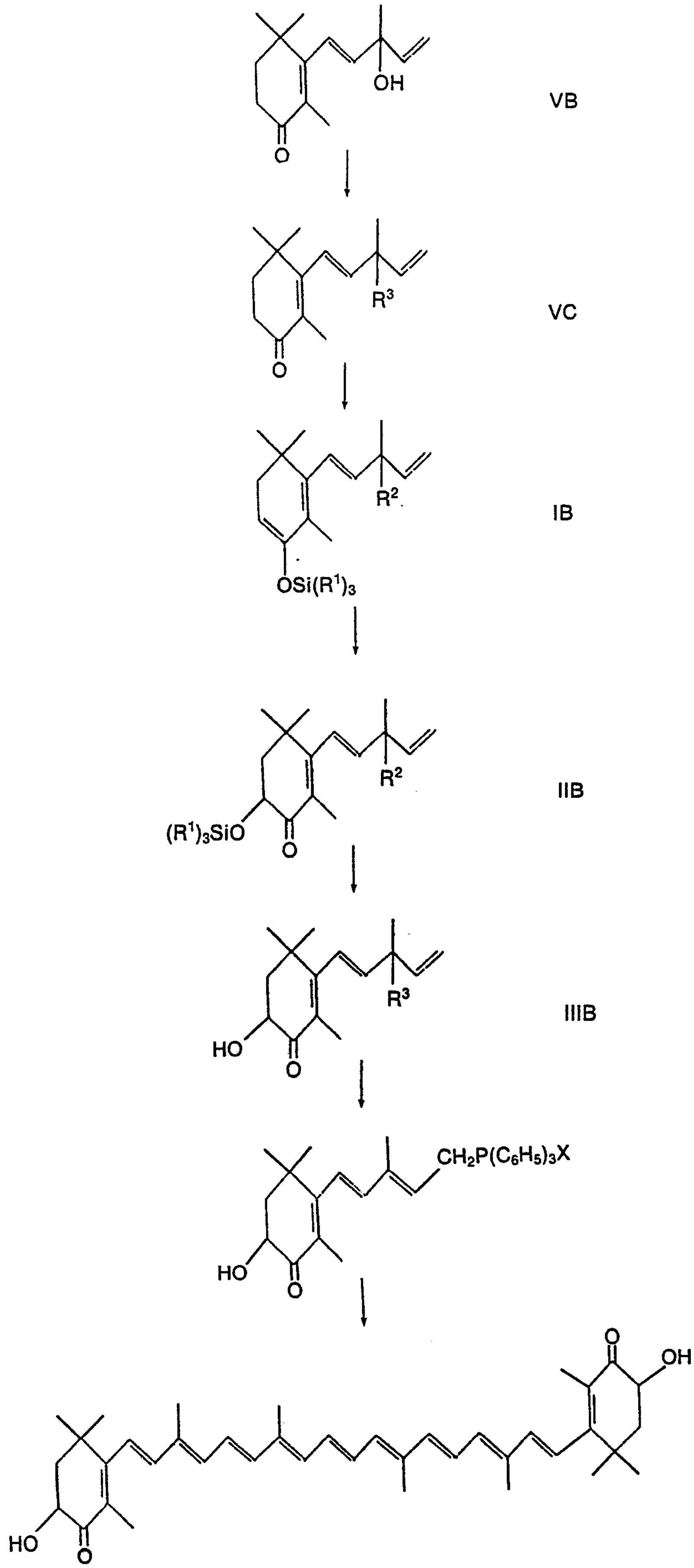

VB

VC

IB

IIB

IIIB

VII

IIIA

Die Verbindungen der Formel VC, worin $R^3$ eine Äthergruppe bedeutet, und die Verbindungen der Formel IIIB sind neu und bilden ebenfalls Gegenstand der vorliegenden Erfindung.

Die Erfindung wird durch die folgenden Beispiele weiter veranschaulicht.

Beispiel 1

a) In einem trockenen Sulfierkolben mit Rührer, Thermometer, Tropftrichter und Argonbegasung wurden 14 ml Tetrahydrofuran und 1,64 ml Diisopropylamin vorgelegt und mit einem Aceton/Trockeneis-Bad auf −15 °C gekühlt. Anschliessend wurden mit dem Tropftrichter 9,5 ml einer Lösung von Butyllithium in Hexan [Gehalt an Butyllithium 1,90M (18,1 mMol)] zum Reaktionsgemisch so zugetropft, dass die Temperatur bei −15 °C gehalten werden konnte, und 15 Minuten bei dieser Temperatur nachgerührt.

b) In einen trockenen Sulfierkolben mit Rührer, Thermometer, Argonbegasung und Kanülenaufsatz wurden 2,83 g (5 mMol) frisch umkristallisiertes Canthaxanthin mit 3 ml (23,7 mMol) Trimethylchlorsilan in 100 ml Tetrahydrofuran vorgelegt und mittels eines Aceton/Trockeneis-Bades auf −15 °C bis −20 °C abgekühlt. Die gemäss Absatz a) hergestellte Lösung von Lithiumdiisopropylamid wurde vollständig in eine gasdichte Dosierspritze transferiert und innert 30 Minuten bei −15 °C mit einer Dosierpumpe zum Reaktionsgemisch gegeben. Nach 5 Minuten Ausrühren wurde der Ansatz mit 200 ml Diäthyläther in einen Scheidetrichter gespült und unter Zugabe von Eis zweimal mit je 200 ml Phosphatpufferlösung (enthaltend 6,8 g Kaliumdihydrogenphosphat und 5,6 ml 1N Natronlauge pro Liter Lösung; pH 6) und dann zweimal mit je 200 ml Eiswasser ausgeschüttelt. Bei jedem Waschgang wurde der Anteil an Tetrahydrofuran, der sich in der Wasserphase löste, wieder zugefügt. Die wässrigen Phasen wurden einmal mit 50 ml Diäthyläther zurückgewaschen. Die organischen Phasen wurden vereinigt, über Natriumsulfat getrocknet, filtriert und am Rotationsverdampfer bei einer Badtemperatur von 50 °C vollständig eingedampft. Hierbei wurden 4,1 g roher Canthaxanthin-bis(trimethylsilyl)enoläther erhalten.

Beispiel 2

a) Der gemäss Beispiel 1 erhaltene Canthaxanthin-bis(trimethylsilyl)enoläther wurde mit 100 ml Diäthyläther in einen Sulfierkolben gespült, der mit Rührer, Thermometer, Tropftrichter und Argonbegasung ausgerüstet ist. Dann wurden 1 g Natriumacetat sowie eine Spatelspitze Sequestren zugegeben und die Suspension mittels eines Aceton/Trockeneis-Bades auf −15 °C abgekühlt. Mittels eines Tropftrichters wurde innert 15 Minuten bei −15 °C eine Lösung von 2,9 g (16 mMol) Monoperphthalsäure in ca. 43 ml Diäthyläther (hergestellt durch Verdünnen der nachstehend beschriebenen Monoperphthalsäurelösung mit Diäthyläther) zum Reaktionsgemisch zugetropft, anschliessend die Temperatur auf 25 °C erhöht und 45 Minuten nachgerührt. Dann wurde das Reaktionsgemisch mit 200 ml Tetrahydrofuran in einen Scheidetrichter gespült und je einmal mit 50 ml 0,5M Natriumpyrosulfit-Lösung, 50 ml einer halbgesättigten Natriumbicarbonat-Lösung und 50 ml einer halbgesättigten Kochsalzlösung ausgeschüttelt. Die wässrigen Phasen wurden einmal mit 50 ml Diäthyläther zurückgewaschen. Die organischen Phasen wurden vereinigt, über Natriumsulfat getrocknet, filtriert und am Rotationsverdampfer bei einer Badtemperatur von 50 °C vollständig eingedampft. Hierbei wurden 4,3 g roher Astaxanthin-bis(trimethylsilyl)-äther erhalten.

b) Der erhaltene Astaxanthin-bis(trimethylsilyl)äther wurde mit 80 ml Methanol in einen Rundkolben mit Rückflusskühler transferiert, mit einer Spatelspitze p-Toluolsulfonsäure versetzt und 30 Minuten am Rückfluss erhitzt. Anschliessend wurde das Methanol am Rotationsverdampfer bei einer Badtemperatur von 50 °C vollständig abgedampft. Das gesamte Rohprodukt wurde in Methylenchlorid gelöst und mittels Hochdruck-Flüssigchromatographie untersucht. Die Ausbeute bezogen auf eingesetztes Canthaxanthin betrug 67,5% Astaxanthin und 2,5% Adonirubin. Ferner wurden 5% nicht umgesetztes Canthaxanthin gefunden.

Die oben verwendete Monoperphthalsäurelösung wurde wie folgt hergestellt:

In einem Sulfierkolben mit Rührer, Thermometer und Tropftrichter wurden 300 ml entionisiertes Wasser, 2,5 g Magnesiumsulfat und 30 g Natriumhydroxid vorgelegt und mit einem Eisbad auf 5 °C abgekühlt. Zu diesem Gemisch wurden innert ca. 2 Minuten 75 ml 30%-iges Wasserstoffperoxid zugetropft, wobei die Temperatur auf 10 °C anstieg, und dann bei 5–10 °C innert 15 Minuten eine Lösung von 37,0 g Phthalsäureanhydrid in 230 ml Tetrahydrofuran zugetropft. Nach 10 Minuten Ausrühren wurde der Ansatz auf 600 ml 20%-ige Schwefelsäure gegossen, wobei die Temperatur von 0 °C auf 10 °C anstieg. Das Reaktionsgemisch wurde dreimal mit je 250 ml Diäthyläther extrahiert. Die vereinigten Ätherphasen wurden zweimal mit je 200 ml einer 40%-igen Ammoniumsulfat-Lösung nachgewaschen (das letzte Waschwasser wies pH 4 auf). Diese beiden Phasen wurden mit 100 ml Diäthyläther zurückgewaschen. Die vereinigten Ätherphasen wurden über Natriumsulfat getrocknet, filtriert und am Rotationsverdampfer bei Raumtemperatur ohne Vakuum auf ein Volumen von ca. 400 ml eingeengt. Die nun ca. 10%-ige Monoperphthalsäurelösung wurde in einen Vierhalskolben gegossen und durch Einblasen von Stickstoff unter Rühren auf ein Volumen von ca. 130 ml eingeengt. Die erhaltene Lösung war ca. 30%-ig (jodometrisch bestimmt). Die chemische Ausbeute an Monoperphthalsäure betrug 88% bezogen auf Phthalsäureanhydrid.

Beispiel 3

In einem trockenen Sulfierkolben mit Tropftrichter, Thermometer, Septum und Argonbegasung wurde unter Rühren eine Lösung von 3,5 ml Diisopropylamin in 30 ml Tetrahydrofuran bei −15 °C innert 5 Minuten tropfenweise (mittels einer Spritze) mit 12 ml einer ca. 2M Lösung von Butyllithium in Hexan versetzt. Das Gemisch wurde noch 15 Minuten bei −15 °C nachgerührt. Anschliessend wurde bei −15 °C bis −20 °C innert ca. 20 Minuten eine Lösung von 5,65 g 96%-igem Canthaxanthin in 400 ml Tetrahydrofuran zugetropft und die braunviolette Suspension 10 Minuten nachgerührt. Dann wurde bei −15 °C eine Lösung von 4 ml Trimethylchlorsilan in 4 ml Tetrahydrofuran zum Reaktionsgemisch zugetropft, das Kühlbad entfernt und die rote Lösung noch 30 Minuten nachgerührt. Das Reaktionsgemisch wurde in einem Scheidetrichter auf 200 ml Diäthyläther und 200 ml Phosphatpufferlösung pH 6 (wie in Beispiel 1) gegossen und ausgeschüttelt. Die organische Phase wurde nochmals mit 200 ml Phosphatpufferlösung pH 6 ausgeschüttelt, anschliessend dreimal mit je 200 ml Wasser gewaschen, über Natriumsulfat getrocknet und am Rotationsverdampfer bei 30 °C auf ein Volumen von ca. 100 ml eingeengt. Diese Lösung von Canthaxanthin-bis(trimethylsilyl)-enoläther wurde direkt zur Weiterreaktion verwendet. Durch Umkristallisation einer Probe aus einem Diäthyläther/Pentan/Methanol-Gemisch wurde Canthaxanthin-bis(trimethylsilyl)-enoläther in Form violetter Nadeln erhalten; Smp. 168–169 °C; Rf-Werte (Diäthyläther/Hexan 2:1): Canthaxanthin-bis(trimethylsilyl)-enoläther ca. 0,75, Canthaxanthin-trimethylsilyl-enoläther ca. 0,54, Canthaxanthin ca. 0,30.

Beispiel 4

a) In einem Sulfierkolben mit Tropftrichter, Thermometer und Argonbegasung wurde unter Rühren die in Beispiel 3 erhaltene Lösung von Canthaxanthin-bis(trimethylsilyl)-enoläther mit 2 g Natriumacetat und 4 g Natriumsulfat versetzt und auf −15 °C gekühlt. Zu diesem Gemisch wurde innert etwa 5 Minuten eine Lösung von 3,4 ml einer ca. 40%-igen Peressigsäure (Konzentration 7,15M gemäss jodometrischer Titration) in 34 ml Diäthyläther zugetropft, dann das Kühlbad entfernt und ca. 2 Stunden nachgerührt. Das Reaktionsgemisch wurde in einem Scheidetrichter einmal mit 100 ml 0,5M eisgekühlter, wässriger Natriumpyrosulfit-Lösung, zweimal mit je 100 ml halbgesättigter, wässriger Natriumhydrogencarbonat-Lösung und einmal mit 100 ml halbgesättigter, wässriger Kochsalzlösung ausgeschüttelt, über Natriumsulfat getrocknet und eingedampft, wobei ca. 8 g dunkelrotes, festes Rohprodukt von Astaxanthin-bis(trimethylsilyl)-äther erhalten wurden; Rf-Wert ca. 0,67 (Diäthyläther/Hexan 2:1). Durch Kristallisation einer Probe aus Methylenchlorid/Aceton wurde der Astaxanthindisilyläther in Form violetter Plättchen erhalten; Smp. 196–197 °C.

b) Das Rohprodukt des Astaxanthin-bis(trimethylsilyl)-äthers wurde in 20 ml Methanol aufgenommen und 4 Stunden unter Argon am Rückfluss gekocht. Das Gemisch wurde auf Raumtemperatur abkühlen gelassen und dann auf −20 °C gekühlt, wobei eine rotviolette Fällung von 4,5 g rohem Astaxanthin erhalten wurde. Einengen der Mutterlauge lieferte ca. 3 g festen, roten Rückstand, aus welchem durch Chromatographie an Kieselgel mit Methylenchlorid/Diäthyläther 9:1 als Eluens und unter einem Argondruck von 0,2–0,4 bar weitere 0,4 g rohes Astaxanthin gewonnen wurden. Das erhaltene, rohe Astaxanthin (4,9 g) wurde in 10 ml Methylenchlorid gelöst und an Kieselgel mit Methylenchlorid/Diäthyläther 9:1 als Eluens und einem Argondruck von 0,2–0,4 bar chromatographiert. Die einheitlichen Fraktionen wurden eingeengt und aus Methylenchlorid/Methanol umkristallisiert, wobei 2,87 g all-trans Astaxanthin (Reinheit ca. 90%) erhalten wurden; Smp. 216–219 °C; Ausbeute 48% bezogen auf Canthaxanthin.

Beispiel 5

Eine Lösung von 565 mg Canthaxanthin in 20 ml Methylenchlorid wurde mit 0,56 ml Triäthylamin versetzt und mit einem Eisbad auf ca. 3 °C abgekühlt. Dann wurden 0,57 ml Trifluormethansulfonsäure-t-butyl-dimethylsilylester zugetropft und das Reaktionsgemisch ohne Kühlung noch 30 Minuten weitergerührt. Anschliessend wurde das Reaktionsgemisch auf ein Gemisch von Eis und gesättigter Kochsalzlösung gegossen und mit Diäthyläther extrahiert. Die Ätherphase wurde dreimal mit Wasser gewaschen, über Natriumsulfat getrocknet und eingeengt. Der erhaltene rohe Canthaxanthin-bis(t-butyl-dimethylsilyl)enoläther kann ohne weitere Reinigung direkt in analoger Weise zu Beispiel 2 oder 4 weiter umgesetzt werden. Durch vollständiges Eindampfen des rohen Enoläthers wurde ein dunkelroter Rückstand erhalten, welcher nach Umkristallisation aus Diäthyläther/Methanol in 70%-iger Ausbeute Canthaxanthin-bis(t-butyl-dimethylsilyl)enoläther in Form feiner, violetter Nadeln lieferte; Smp. 166–168 °C.

Beispiel 6

In einem Vierhalskolben mit Rührer und Rückflusskühler wurde eine Lösung von 23,1 g 96,5%-igem
1-(3-Oxo-2,6,6-trimethyl-1-cyclohexen-1-yl)-3-methyl-1,4-pentadien-3-ol
in 85 ml Dimethylformamid mit 80 ml Triäthylamin und 73 ml Trimethylchlorsilan versetzt. Das Gemisch wurde unter Rühren und einem leichten Argonstrom während 2 Stunden zum Rückfluss erhitzt (Heizbadtemperatur 120 °C). Anschliessend wurde das Reaktionsgemisch mit einem Eisbad auf ca. 0 °C abgekühlt und unter Rühren mit 400 ml Hexan und 200 ml halbgesättigter wässriger Natriumhydrogencarbonat-Lösung versetzt. Die Phasen wurden getrennt und die wässrige Phase nochmals mit 200 ml Hexan extrahiert. Die vereinigten organischen Phasen wurden einmal mit 100 ml gesättigter Kochsalzlösung gewaschen, über Natriumsulfat getrocknet und im Rotationsverdampfer bei 40 °C eingeengt. Destillation des erhaltenen braunen Öles (39,5 g) bei 102–107 °C/0,01 Torr ergab 35,0 g
Trimethyl[[1-methyl-3-[2,6,6-trimethyl-3-(trimethylsiloxy)-1,3-cyclohexadien-1-yl]-1-vinylallyl]oxy]silan
als leicht gelbliches Öl; Reinheit ca. 77%.

Beispiel 7

a) In einem Vierhalskolben mit Rührer wurden unter Argon 18,3 g ca. 40%-ige Peressigsäure, 190 ml absolutes Methylenchlorid, 3,8 g wasserfreies Magnesiumsulfat und 2,2 g wasserfreies Natriumacetat vorgelegt und unter Rühren auf −15 °C gekühlt. Zu dieser Aufschlämmung wurde innert 10 Minuten eine Lösung von 35 g 77%-igem

Trimethyl[[1-methyl-3-[2,6,6-trimethyl-3-(trimethylsiloxy)-1,3-cyclohexadien-1-yl]-1-vinylallyl]oxy]silan

(hergestellt nach Beispiel 6) in 70 ml Methylenchlorid zugetropft. Das Eisbad wurde entfernt und das Gemisch weitere 15 Minuten gerührt, wobei die Temperatur auf 17 °C anstieg. Das Reaktionsgemisch wurde unter Rühren mit 38 g wasserfreiem Natriumcarbonat und 38 g Natriumhydrogencarbonat versetzt, dann 20 Minuten weitergerührt und filtriert (Nachwaschen mit 150 ml Methylenchlorid). Das erhaltene Filtrat (ca. 400 ml) enthaltend

Trimethyl[[1-methyl-3-[2,6,6-trimethyl-3-oxo-4-(trimethylsiloxy)-1-cyclohexen-1-yl]-1-vinylallyl]oxy]silan

wurde direkt weiter umgesetzt.

b) Das erhaltene Filtrat wurde unter starkem Rühren bei Raumtemperatur mit 40 ml Triäthylammoniumfluorid versetzt und dann noch 20 Stunden gerührt. Die Reaktionslösung wurde nacheinander mit 100 ml halbgesättigter Kochsalzlösung, 100 ml halbgesättigter wässriger Natriumpyrosulfitlösung und nochmals mit 100 ml halbgesättigter Kochsalzlösung gewaschen, über Natriumsulfat getrocknet und im Rotationsverdampfer bei 40 °C Badtemperatur eingeengt. Hierbei wurden 25,3 g

1-(4-Hydroxy-3-oxo-2,6,6-trimethyl-1-cyclohexen-1-yl)-3-methyl-1,4-pentadien-3-ol

als gelbliches Öl erhalten; Reinheit 79,8% gemäss Gaschromatographie.

c) Das erhaltene

1-(4-Hydroxy-3-oxo-2,6,6-trimethyl-1-cyclohexen-1-yl)-3-methyl-1,4-pentadien-3-ol

wurde in 250 ml Methylenchlorid mit 30 ml 63%-iger Bromwasserstofflösung zum Bromid und anschliessend mit 26,2 g Triphenylphosphin zum Phosphoniumbromid umgesetzt. Hierbei wurden 45,3 g rohes

[(4E)-5-(4-Hydroxy-2,6,6-trimethyl-3-oxo-1-cyclohexen-1-yl)-3-methyl-2,4-pentadienyl]-triphenylphosphoniumbromid

erhalten; Smp. 157–163 °C. Nach Umkristallisation aus Essigester/Methylenchlorid 2:1 bei 0 °C wurden 32,4 g 89,9%-iges trans-Produkt erhalten; Smp. 181–182 °C. Nach zwei weiteren Umkristallisationen aus Essigester/Methylenchlorid betrug die Reinheit 96%; Smp. 186–187 °C.

Beispiel 8

In einem Vierhalskolben mit Rührer wurden 22,2 g Diisopropylamin in 330 ml absolutem Tetrahydrofuran gelöst und unter Argon auf −15 °C gekühlt. Zu dieser Lösung wurden unter Rühren 106 ml einer ca. 2M Lösung von Butyllithium in Hexan und nach 10 Minuten eine Lösung von 23,2 g 97%-igem

1-(3-Oxo-2,6,6-trimethyl-1-cyclohexen-1-yl)-3-methyl-1,4-pentadien-3-ol

in 40 ml absolutem Tetrahydrofuran zugetropft. Anschliessend wurden 48 ml Trimethylchlorsilan so zugetropft, dass die Reaktionstemperatur auf −15 °C gehalten werden konnte. Das Reaktionsgemisch wurde hierbei wieder homogen. Nach der Zugabe wurde das Kühlbad entfernt und das Reaktionsgemisch auf Raumtemperatur erwärmen gelassen. Das Reaktionsgemisch wurde in einen 2 l-Kolben transferiert (Nachwaschen mit Pentan) und das Lösungsmittel am Rotationsverdampfer unter Vakuum bei ca. 40 °C Badtemperatur entfernt. Der Rückstand wurde in ca. 250 ml Pentan aufgenommen, die erhaltene Suspension filtriert und der Filterrückstand mehrmals mit Pentan (total 250 ml) nachgewaschen. Das Filtrat wurde im Rotationsverdampfer eingedampft und das erhaltene Rohprodukt (43,3 g) bei 110 °C/0,01 Torr destilliert. Hierbei wurden 37,2 g

Trimethyl[[1-methyl-3-[2,6,6-trimethyl-3-(trimethylsiloxy)-1,3-cyclohexadien-1-yl]-1-vinylallyl]oxy]silan

in einer Reinheit von 98% als leicht gelbliches Öl erhalten.

Beispiel 9

a) 37,2 g

Trimethyl[[1-methyl-3-[2,6,6-trimethyl-3-(trimethylsiloxy)-1,3-cyclohexadien-1-yl]-1-vinylallyl]oxy]silan (Reinheit 98%)

wurden in 250 ml absolutem Diäthyläther gelöst und unter Argon und gutem Rühren mit 24,7 g Natriumhydrogencarbonat, 31,2 g Natriumcarbonat und 24,4 g Natriumacetat versetzt. Das Reaktionsgemisch wurde 10 Minuten bei −5 °C gerührt und dann innert 20 Minuten mit 326 ml einer 0,9N Lösung von Monoperphthalsäure in Diäthyläther versetzt. Das Reaktionsgemisch wurde auf 100 ml gesättigte Natriumhydrogencarbonat-Lösung gegossen und mit einem Gemisch von 500 ml Wasser und 500 ml Diäthyläther extrahiert. Die organische Phase wurde einmal mit 200 ml halbgesättigter Natriumpyrosulfit-Lösung und zweimal mit je 100 ml Kochsalzlösung gewaschen und dann eingedampft. Der Eindampfrückstand (41,2 g) von

Trimethyl[[1-methyl-3-[2,6,6-trimethyl-3-oxo-4-(trimethylsiloxy-1-cyclohexen-1-yl]-1-vinylallyl]oxy]silan

wurde direkt weiter umgesetzt.

b) Der Eindampfrückstand wurde in 200 ml Methylenchlorid gelöst und dann mit 40 ml Triäthylammoniumfluorid-Lösung versetzt und 4 Stunden bei Raumtemperatur gerührt. Anschliessend wurde das Reaktionsgemisch auf 100 ml halbgesättigte Natriumhydrogencarbonat-Lösung gegossen und dann die organische Phase abgetrennt und eingedampft. Hierbei wurden 28,0 g Eindampfrückstand von

1-(4-Hydroxy-3-oxo-2,6,6-trimethyl-1-cyclohexen-1-yl)-3-methyl-1,4-pentadien-3-ol

als Öl erhalten, welches direkt weiter umgesetzt wurde.

c) Das erhaltene Öl wurde unter Argon bei 0 °C in 250 ml absolutem Methylenchlorid aufgenommen und innert 10 Minuten mit 30 ml 63%-iger Bromwasserstofflösung versetzt. Das Reaktionsgemisch wurde in ca. 0,5 l Essigester aufgenommen, einmal mit gesättigter Kochsalzlösung und einmal mit gesättigter Natriumhydrogencarbonat-Lösung gewaschen, über Natriumsulfat getrocknet und im Rotationsverdampfer bei 30 °C Badtemperatur auf ca. 300 ml eingeengt. Die so erhaltene Bromid-Lösung wurde zu einer Lösung von 26,2 g Triphenylphosphin in 100 ml absolutem Essigester getropft und über Nacht bei Raumtemperatur gerührt. Nach Filtration wurden 49,0 g rohes
[5-(4-Hydroxy-2,6,6-trimethyl-3-oxo-1-cyclohexen-1-yl)-3-methyl-2,4-pentadienyl]-triphenylphosphoniumbromid
(Gehalt gemäss Hochdruck-Flüssigchromatographie: 77,2% 2E- und 12,1% 2Z-Isomer) erhalten; Smp. 162–164 °C. Umkristallisation aus 200 ml Methylenchlorid und 400 ml Essigester ergab 43,2 g Phosphoniumsalz (Gehalt: 84,5% 2E- und 10% 2Z-Isomer) mit Smp. 178–180 °C.

Beispiel 10

a) 2,34 g
1-(3-Oxo-2,6,6-trimethyl-1-cyclohexen-1-yl)-3-methyl-1,4-pentadien-3-ol
wurden in 15 ml Hexan und 5 ml Diäthyläther gelöst, mit 2 Tropfen Tricaprylmethylammoniumchlorid und 4,54 ml wässriger Kaliumhydroxid-Lösung (hergestellt aus 70 g pulverförmigem Kaliumhydroxid und 50 ml Wasser) versetzt und gut gerührt. Anschliessend wurden 1,98 g Dimethylsulfat zum Reaktionsgemisch zugetropft und die Reaktion mittels Gaschromatographie verfolgt. Nach 4-stündigem Rühren bei Raumtemperatur (Produkt/Edukt-Verhältnis 99,7:0,3) wurde das Gemisch auf Eis gegossen. Die organische Phase wurde abgetrennt, mit Wasser neutral gewaschen, über Natriumsulfat getrocknet und am Wasserstrahlvakuum eingeengt. Es resultierten 2,47 g
1-(3-Oxo-2,6,6-trimethyl-1-cyclohexen-1-yl)-3-methoxy-3-methyl-1,4-pentadien
als gelbes Öl.

b) Eine Lösung von 19,5 g Diisopropylamin in 300 ml absolutem Tetrahydrofuran wurde auf −20 °C abgekühlt, tropfenweise mit 126 ml einer 1,6 M Lösung von Butyllithium in Hexan versetzt und noch 20 Minuten bei −20 °C gerührt. Dann wurde das Gemisch tropfenweise mit einer Lösung von 40,0 g
1-(3-Oxo-2,6,6-trimethyl-1-cyclohexen-1-yl)-3-methoxy-3-methyl-1,4-pentadien
in 50 ml absolutem Tetrahydrofuran versetzt und noch 1 Stunde bei 0 °C gerührt. Nach erneutem Abkühlen auf −20 °C und raschem Zutropfen von 21 g Trimethylchlorsilan wurde das Reaktionsgemisch nochmals 15 Minuten bei 0 °C gerührt (gemäss Gaschromatographie waren zu diesem Zeitpunkt 96,5% des Edukts zum gewünschten Produkt umgesetzt). Das Reaktionsgemisch wurde tropfenweise mit 80 ml gesättigter Kochsalzlösung versetzt und dann mit Diäthyläther extrahiert. Die organische Phase wurde dreimal mit gesättigter Kochsalzlösung gewaschen, über Natriumsulfat getrocknet und am Wasserstrahlvakuum eingeengt. Es wurden 51,0 g
1-[2-6,6-Trimethyl-3-(trimethylsiloxy)-1,3-cyclohexadien-1-yl]-3-methoxy-3-methyl-1,4-pentadien (Reinheit 96,36%)
erhalten.

Beispiel 11

a) Ein Gemisch von 32,0 g
1-[2,6,6-Trimethyl-3-(trimethylsiloxy)-1,3-cyclohexadien-1-yl]-3-methoxy-3-methyl-1,4-pentadien,
10,0 g Natriumhydrogencarbonat, 10,0 g Magnesiumsulfat-trihydrat und 500 ml Diäthyläther wurde bei 2–5 °C gut gerührt und dann tropfenweise mit 26,45 g 40%-iger Peressigsäure versetzt und auf Raumtemperatur erwärmen gelassen. Anschliessend wurde das Reaktionsgemisch auf 0 °C abgekühlt, tropfenweise mit 90 ml eines Gemisches von Methanol und 1N Salzsäure (Vol. 1:1) versetzt und mit Diäthyläther extrahiert. Die organische Phase wurde zweimal mit kalter, halbkonzentrierter Natriumpyrosulfit-Lösung, einmal mit Eiswasser, zweimal mit Eiswasser, welchem eine kleine Menge gesättigte Natriumhydrogencarbonat-Lösung zugesetzt worden war, und noch zweimal mit Eiswasser gewaschen. Nach dem Trocknen über Natriumsulfat und dem Einengen am Wasserstrahlvakuum bei 35 °C wurden 26,4 g rohes
1-(4-Hydroxy-3-oxo-2,6,6-trimethyl-1-cyclohexen-1-yl)-3-methoxy-3-methyl-1,4-pentadien
(Reinheit 91,5%) erhalten, welches noch 7,07%
1-(3-Oxo-2,6,6-trimethyl-1-cyclohexen-1-yl)-3-methoxy-3-methyl-1,4-pentadien
enthielt.

b) Eine Lösung von 58,0 g rohem, 91,55%-igem
1-(4-Hydroxy-3-oxo-2,6,6-trimethyl-1-cyclohexen-1-yl)-3-methoxy-3-methyl-1,4-pentadien
in 400 ml Methylenchlorid wurde bei −20 °C gerührt, durch rasches Zutropfen mit 38,5 g 63%-iger wässriger Bromwasserstoff-Lösung versetzt und anschliessend noch 10 Minuten bei −15 °C gerührt. Das Reaktionsgemisch wurde mit kaltem Essigsäureäthylester extrahiert. Der Extrakt wurde mit kalter, 25%-iger wässriger Natriumbromid-Lösung und wenig gesättigter Natriumhydrogencarbonat-Lösung gewaschen und dann am Wasserstrahlvakuum bei Raumtemperatur auf ein Volumen von ca. 70–80 ml eingeengt. Die erhaltene Lösung wurde zu einer Lösung von 52,4 g Triphenylphosphin in 200 ml Essigsäureäthylester gegeben und das Gemisch ca. 3–4 Stunden bei Raumtemperatur gerührt. Nach Kristallisation des Produktes wurde das Reaktionsgemisch noch 15 Stunden bei 5 °C gerührt, dann genutscht und das Nutschgut bei 35 °C am Wasserstrahlvakuum getrocknet. Das erhaltene rohe Phosphoniumsalz (95,3 g) wurde aus 200 ml Methylenchlorid und 400 ml Essigsäureäthylester umkristallisiert. Hierbei wurden 80,35 g
[(4E)-5-(4-Hydroxy-2,6,6-trimethyl-3-oxo-1-cyclohexen-1-yl)-3-methyl-2,4-pentadienyl]-triphenylphosphoniumbromid
(enthaltend 83,36% 2E- und 13,55% 2Z-Isomer) mit Smp. 179–182 °C erhalten.

**Patentansprüche**

1. Verfahren zur Herstellung von Cyclohexenonderivaten, dadurch gekennzeichnet, dass man einen Silyl-enoläther der allgemeinen Formel

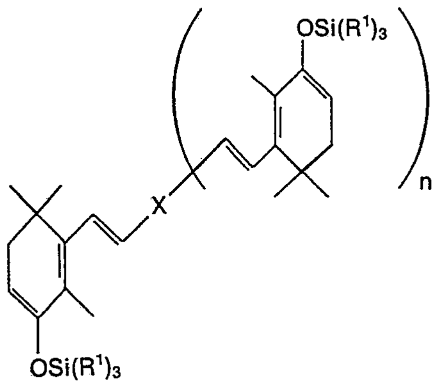

I

worin n für die Zahl 0 und X für eine Gruppe

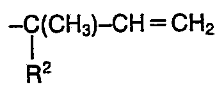

oder n für die Zahl 1 und X für die Gruppe 6,11-Dimethylhexadeca-2,4,6,8,10,12,14-heptaen-2,15-diyl stehen, die Reste $R^1$ gleiche oder verschiedene Bedeutung haben und Alkylgruppen bezeichnen, und der Rest $R^2$ eine Trialkylsiloxygruppe $-OSi(R^1)_3$ oder eine Äthergruppe darstellt, mit Percarbonsäure oxidiert und, gewünschtenfalls, das erhaltene α-Siloxyketon der allgemeinen Formel

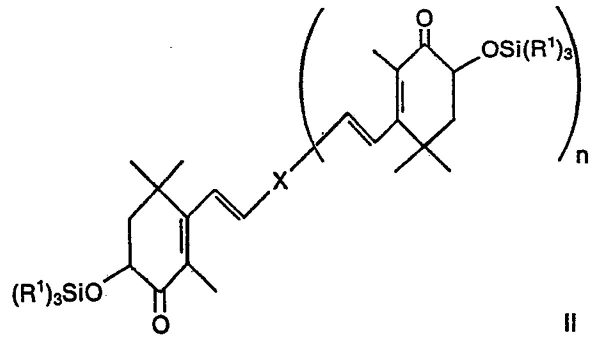

II

worin n, X und $R^1$ die obigen Bedeutungen haben, zu einem α-Hydroxyketon der allgemeinen Formel

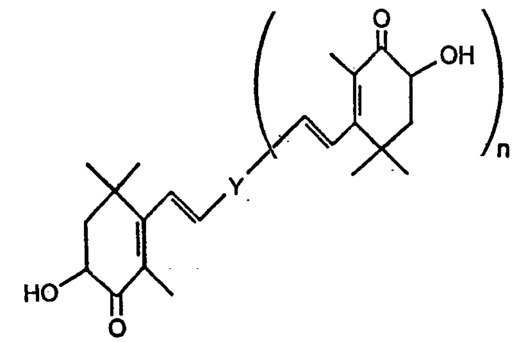

III

worin n für die Zahl 0 und Y für eine Gruppe

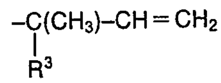

oder n für die Zahl 1 und Y für die Gruppe 6,11-Dimethylhexadeca-2,4,6,8,10,12,14-heptaen-2,15-diyl stehen, und $R^3$ die Hydroxygruppe oder eine Äthergruppe darstellt, hydrolysiert.

2. Verfahren nach Anspruch 1, dadurch gekennzeichnet, dass man eine Verbindung der allgemeinen Formel

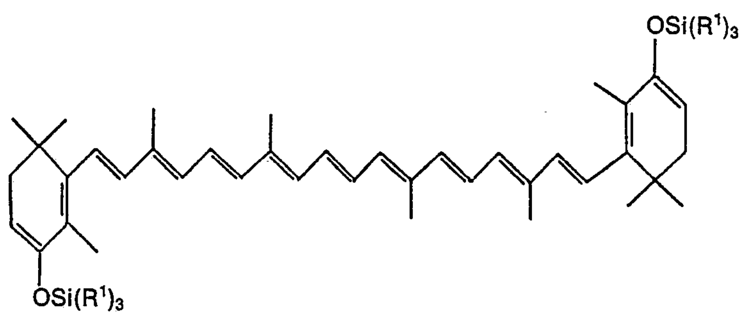

IA

worin die Reste $R^1$ die in Anspruch 1 gegebene Bedeutung haben, umsetzt.

3. Verfahren nach Anspruch 1, dadurch gekennzeichnet, dass man eine Verbindung der allgemeinen Formel

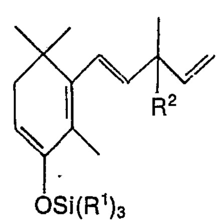

IB

worin die Reste $R^1$ und $R^2$ die in Anspruch 1 gegebenen Bedeutungen haben, umsetzt.

4. Verfahren nach Anspruch 3, dadurch gekennzeichnet, dass man eine Verbindung der Formel IB umsetzt, worin $R^2$ eine Alkoxygruppe mit 1 bis 5 Kohlenstoffatomen, vorzugsweise Methoxy bedeutet.

5. Verfahren nach Anspruch 3, dadurch gekennzeichnet, dass man eine Verbindung der allgemeinen Formel

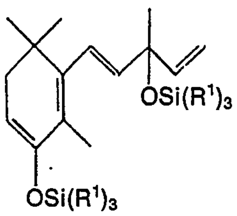

IC

worin die Reste $R^1$ die in Anspruch 1 gegebene Bedeutung haben, umsetzt.

6. Verfahren nach einem der Ansprüche 1 bis 5, dadurch gekennzeichnet, dass man eine Verbindung der Formel I umsetzt, worin die Reste $R^1$ Alkylgruppen mit 1 bis 5 Kohlenstoffatomen bezeichnen.

7. Verfahren nach Anspruch 6, dadurch gekennzeichnet, dass man eine Verbindung der Formel I umsetzt, worin die Trialkylsiloxygruppe $-OSi(R^1)_3$ Trimethylsiloxy oder t-Butyldimethylsiloxy darstellt.

8. Verfahren nach einem der Ansprüche 1 bis 7, dadurch gekennzeichnet, dass man die Oxidation einer Verbindung der Formel I mit Monoperphthalsäure, Peressigsäure oder Perbenzoesäure durchführt.

9. Verbindungen der allgemeinen Formel

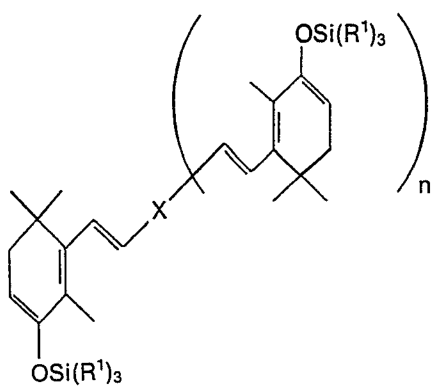

I

worin n für die Zahl 0 und X für eine Gruppe

$$-\underset{R^2}{C}(CH_3)-CH=CH_2$$

oder n für die Zahl 1 und X für die Gruppe 6,11-Dimethylhexadeca-2,4,6,8,10,12,14-heptaen-2,15-diyl stehen, die Reste $R^1$ gleiche oder verschiedene Bedeutung haben und Alkylgruppen bezeichnen, und der Rest $R^2$ eine Trialkylsiloxygruppe $-OSi(R^1)_3$ oder eine Äthergruppe darstellt.

10. Verbindungen der allgemeinen Formel

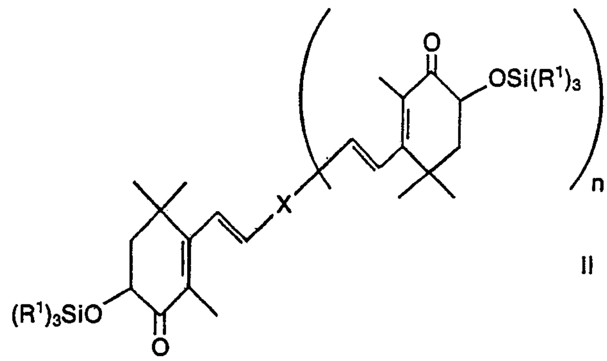

II

worin n für die Zahl 0 und X für eine Gruppe

$$-\underset{R^3}{C}(CH_3)-CH=CH_2$$

oder n für die Zahl 1 und X für die Gruppe 6,11-Dimethylhexadeca-2,4,6,8,10,12,14-heptaen-2,15-diyl

stehen, die Reste $R^1$ gleiche oder verschiedene Bedeutung haben und Alkylgruppen bezeichnen, und der Rest $R^2$ eine Trialkylsiloxygruppe $-OSi(R^1)_3$ oder eine Äthergruppe darstellt.

## Claims

1. A process for the manufacture of cyclohexenone derivatives, characterized by oxidizing a silyl-enol ether of the general formula

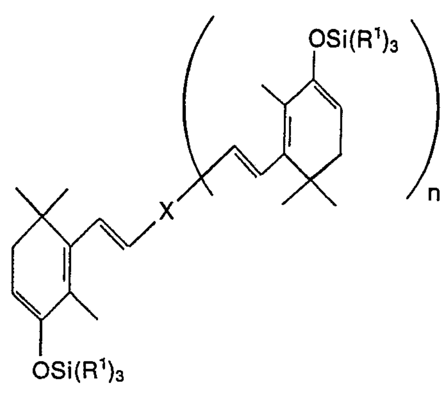

I

wherein n stands for the number 0 and X stands for a group

$$-\underset{R^2}{C}(CH_3)-CH=CH_2$$

or n stands for the number 1 and X stands for the 6,11-dimethylhexadeca-2,4,6,8,10,12,14-heptaene-2,15-diyl group,

the residues $R^1$ have the same or different significances and denote alkyl groups, and the residue $R^2$ represents a trialkylsiloxy group $-OSi(R^1)_3$ or an ether group,

with percarboxylic acid and, if desired, hydrolyzing the resulting α-siloxy ketone of the general formula

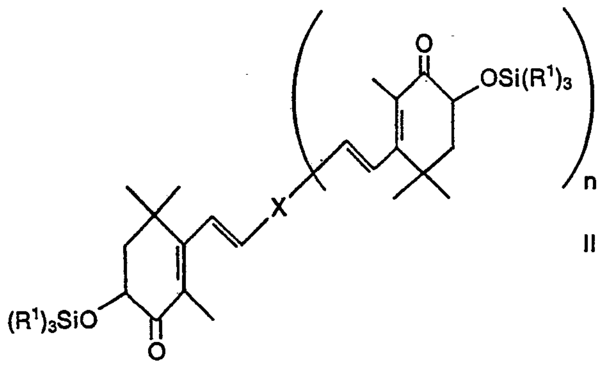

II

wherein n, X and $R^1$ have the above significances, to an α-hydroxy ketone of the general formula

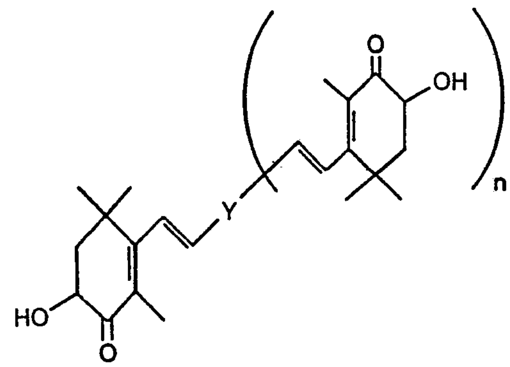

III

wherein n stands for the number 0 and Y stands for a group

$$-\underset{R^3}{\underset{|}{C}}(CH_3)-CH=CH_2$$

or n stands for the number 1 and Y stands for the 6,11-dimethylhexadeca-2,4,6,8,10,12,14-heptaene-2,15-diyl group,

and $R^3$ represents the hydroxy group or an ether group.

2. A process according to claim 1, characterized in that a compound of the general formula

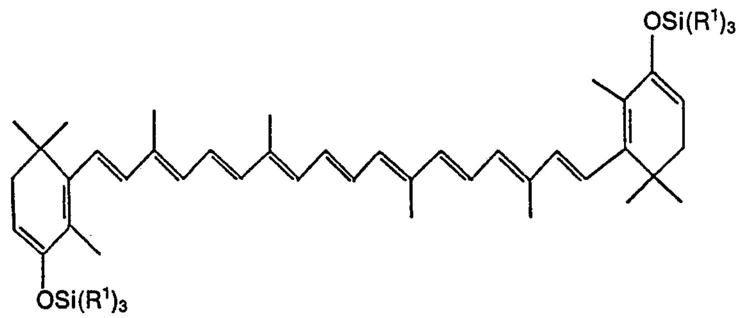

IA

wherein the residues $R^1$ have the significance given in claim 1,
is reacted.

3. A process according to claim 1, characterized in that a compound of the general formula

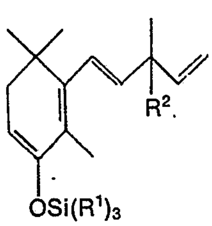

IB

wherein the residues $R^1$ and $R^2$ have the significance given in claim 1,
is reacted.

4. A process according to claim 3, characterized in that a compound of formula IB in which $R^2$ signifies an alkoxy group with 1 to 5 carbon atoms, preferably methoxy, is reacted.

5. A process according to claim 3, characterized in that a compound of the general formula

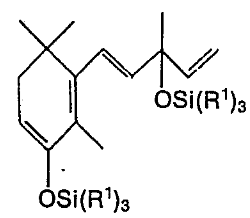

IC

wherein the residues $R^1$ have the significance given in claim 1,
is reacted.

6. A process according to any one of claims 1 to 5, characterized in that a compound of formula I in which the residues $R^1$ denote alkyl groups with 1 to 5 carbon atoms is reacted.

7. A process according to claim 6, characterized in that a compound of formula I in which the trialkylsiloxy group $-OSi(R^1)_3$ represents trimethylsiloxy or t-butyl-dimethylsiloxy is reacted.

8. A process according to any one of claims 1 to 7, characterized in that the oxidation of a compound of formula I is carried out with monoperphthalic acid, peracetic acid or perbenzoic acid.

9. Compounds of the general formula

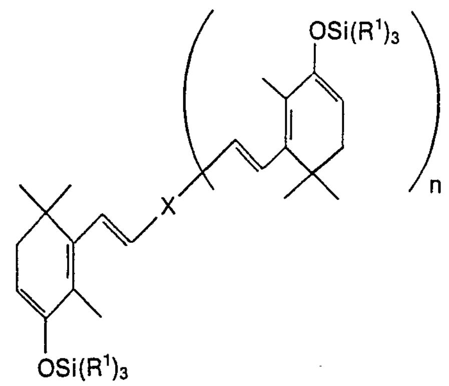

I

wherein n stands for the number 0 and X stands for a group

$-C(CH_3)-CH=CH_2$
|
$R^2$

or n stands for the number 1 and X stands for the 6,11-dimethylhexadeca-2,4,6,8,10,12,14-heptaene-2,15-diyl group,
the residues $R^1$ have the same or different significances and denote alkyl groups, and the residue $R^2$ represents a trialkylsiloxy group $-OSi(R^1)_3$ or an ether group.

10. Compounds of the general formula

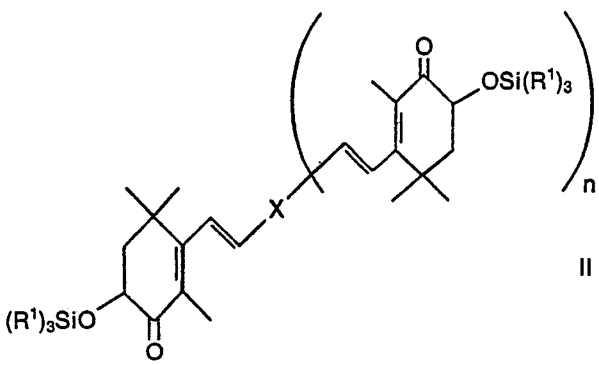

II

wherein n stands for the number 0 and X stands for a group

$-C(CH_3)-CH=CH_2$
|
$R^2$

or n stands for the number 1 and X stands for the 6,11-dimethylhexadeca-2,4,6,8,10,12,14-heptaene-2,15-diyl group,

the residues $R^1$ have the same or different significances and denote alkyl groups, and the residue $R^2$ represents a trialkylsiloxy group $-OSi(R^1)_3$ or an ether group.

## Revendications

1. Procédé de préparation de dérivés de cyclohexénone, caractérisé en ce qu'on oxyde par un acide percarboxylique un silyl-énoléther de formule générale

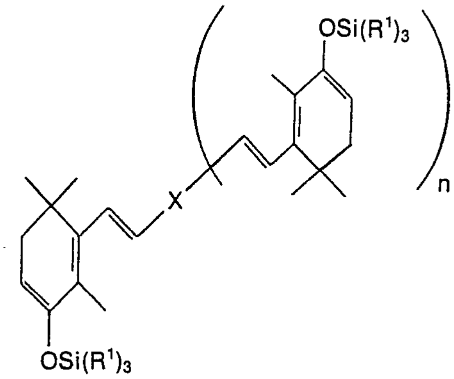

I

dans laquelle n est le nombre 0 et X est un groupe

$-C(CH_3)-CH=CH_2$
|
$R^3$

ou n est le nombre 1 et X est le groupe 6,11-diméthylhexadéca-2,4,6,8,10,12,14-heptaène-2,15-diyle
et les restes $R^1$ ont des significations identiques ou différentes et désignent des groupes alkyles et le reste $R^2$ représente un groupe trialkylsilyloxy $-OSi(R^1)_3$ ou un groupe éther,
et, le cas échéant, on hydrolyse l'α-siloxycétone de formule générale

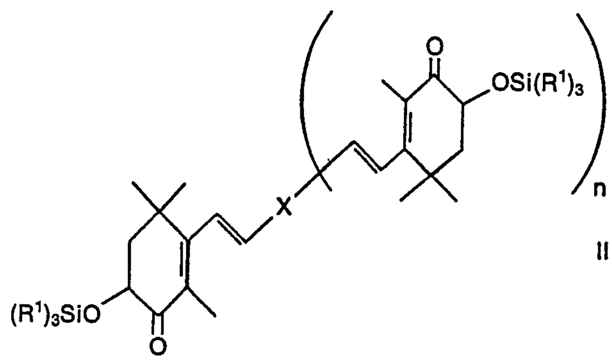

II

dans laquelle n, X et $R^1$ ont les significations ci-dessus,
en une α-hydroxycétone de formule générale

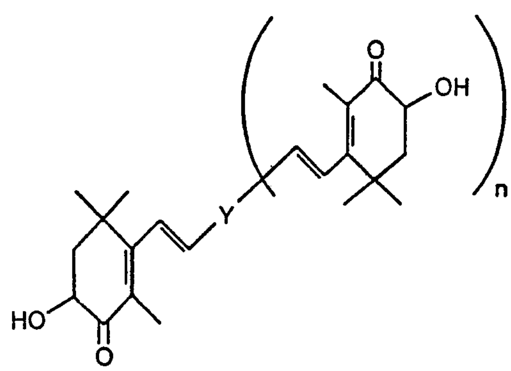

III

dans laquelle n est le nombre 0 et Y est un groupe

$-C(CH_3)-CH=CH_2$
|
$R^2$

ou n est le nombre 1 et Y le groupe
6,11-diméthylhexadéca-2,4,6,8,10,12,14-
heptaène-2,15-diyle
et $R^3$ est le groupe hydroxy ou un groupe éther.

2. Procédé selon la revendication 1, caractérisé en ce qu'on fait réagir un composé de formule générale

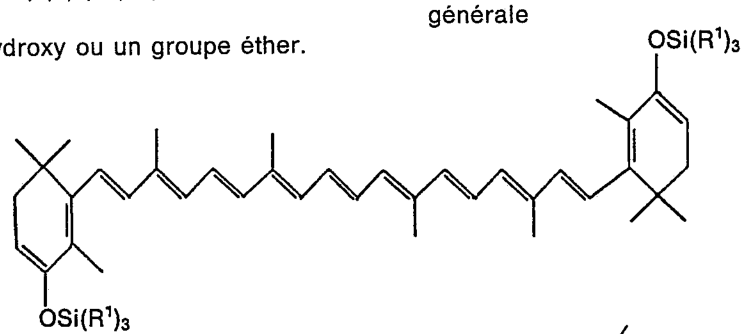

IA

dans lequel les restes $R^1$ ont la signification donnée dans la revendication 1.

3. Procédé selon la revendication 1, caractérisé en ce qu'on fait réagir un composé de formule générale

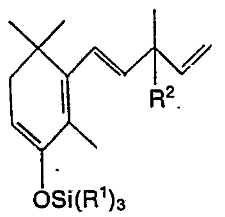

IB

dans laquelle les restes $R^1$ et $R^2$ ont les significations données dans la revendication 1.

4. Procédé selon la revendication 3, caractérisé en ce qu'on fait réagir un composé de formule IB, dans lequel $R^2$ représente un groupe alcoxy avec de 1 à 5 atomes de carbone, de préférence un méthoxy.

5. Procédé selon la revendication 3, caractérisé en ce qu'on fait réagir un composé de formule générale

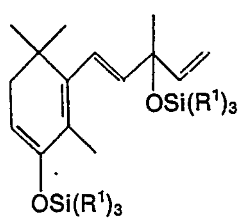

IC

dans laquelle le reste $R^1$ a la signification donnée dans la revendication 1.

6. Procédé selon l'une des revendications 1 à 5, caractérisé en ce qu'on fait réagir un composé de formule I, dans laquelle les restes $R^1$ représentent des groupes alkyle de 1 à 5 atomes de carbone.

7. Procédé selon la revendication 6, caractérisé en ce qu'on fait réagir un composé de formule I, dans laquelle le groupe trialkylsiloxy $-OSi(R^1)_3$ représente le triméthylsiloxy ou le t-butyldiméthylsiloxy.

8. Procédé selon l'une des revendications 1 à 7, caractérisé en ce qu'on réalise l'oxydation d'un composé de formule I avec de l'acide monoperphtalique, de l'acide peracétique ou de l'acide perbenzoïque.

9. Composés de formule générale

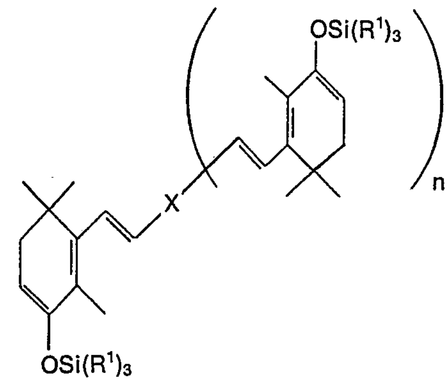

I

dans laquelle n est le nombre 0 et X est un groupe

$$-C(CH_3)(R^2)-CH=CH_2$$

ou n est le nombre 1 et X est le groupe
6,11-diméthylhexadéca-2,4,6,8,10,12,14-
heptaène-2,15-diyle,
et les restes $R^1$ ont des significations identiques ou différentes et désignent des groupes alkyles et le reste $R^2$ représente un groupe trialkylsiloxy $-OSi(R^1)_3$ ou un groupe éther.

10. Composés de formule générale

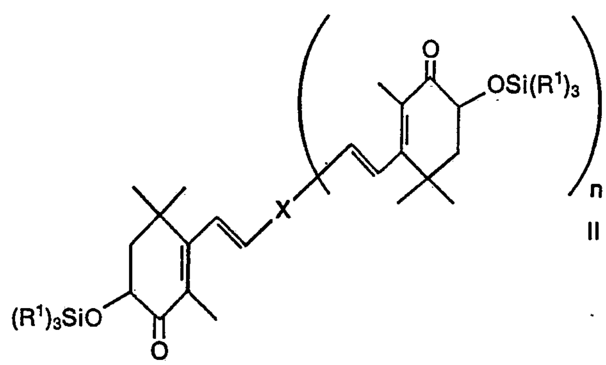

II

dans laquelle n est le nombre 0 et X est un groupe

$$-C(CH_3)(R^2)-CH=CH_2$$

ou n est le nombre 1 et X est le groupe
6,11-diméthylhexadéca-2,4,6,8,10,12,14-
heptaène-2,15-diyle,
les restes $R^1$ ont des significations identiques ou différentes et désignent des groupes alkyles et le reste $R^2$ représente un groupe trialkylsiloxy $-OSi(R^1)_3$ ou un groupe éther.